# EUROPÄISCHE PATENTANMELDUNG

(11) **EP 4 749 636 A1**
(43) Veröffentlichungstag der Anmeldung: **27.05.2026**
(21) Anmeldenummer: 24214823.7
(22) Anmeldetag: 22.11.2024
(51) Int. Cl.: G16H 20/30, G16H 20/40, G16H 40/63

(54) **VERFAHREN ZUR GENERIERUNG EINER BILDDARSTELLUNG BEI EINER MEDIZINISCHEN BILDGEBUNG, STEUERUNGSEINRICHTUNG, WIEDERGABEEINRICHTUNG UND BILDGEBUNGSEINRICHTUNG**

(71) Anmelder: Siemens Healthineers AG, 91301 Forchheim (DE)
(72) Erfinder: Batzer, Ulrich, 91080 Spardorf (DE)
(74) Vertreter: Siemens Healthineers Patent Attorneys

(57) **Zusammenfassung**

Verfahren zur Generierung einer Bilddarstellung (18) für einen Patienten (5) während der Durchführung einer medizinischen Bildgebung mittels einer medizinischen Bildgebungseinrichtung (1), wobei während der Durchführung der Bildgebung Bilddaten (7) betreffend zumindest einen Teil eines Körpers des sich in einem Aufnahmebereich (10) der Bildgebungseinrichtung (1) befindenden Patienten (5) erhoben werden, wobei mittels wenigstens einer Wiedergabeeinrichtung (2) die Bilddarstellung (18) für den Patienten (5) im Aufnahmebereich (10) generiert wird, wobei die Bilddarstellung (18) anhand der im Rahmen der Durchführung der Bildgebung erhobenen Bilddaten (7) erfolgt.

## Beschreibung

Die vorliegende Erfindung betrifft ein Verfahren zur Generierung einer Bilddarstellung für einen Patienten während der Durchführung einer medizinischen Bildgebung mittels einer medizinischen Bildgebungseinrichtung, wobei während der Durchführung der Bildgebung Bilddaten betreffend zumindest einen Teil eines Körpers des sich in einem Aufnahmebereich der Bildgebungseinrichtung befindenden Patienten erhoben werden, wobei mittels wenigstens einer Wiedergabeeinrichtung die Bilddarstellung für den Patienten im Aufnahmebereich generiert wird.

Ein Problem im Zusammenhang mit der Erfassung von Bilddaten während der Durchführung einer medizinischen Bildgebung mittels einer medizinischen Bildgebungseinrichtung, insbesondere einer Magnetresonanztomographieeinrichtung oder einer Computertomographieeinrichtung, ist der Umstand, dass der Patient während der Erhebung der Bilddaten möglichst ruhig und still liegen sollte. Insbesondere da eine solche Bildgebung häufig eine nicht unerhebliche Zeitdauer in Anspruch nimmt, beispielsweise bis zu zwei Stunden, fällt es dem Patienten oft schwer, dies auch tatsächlich umzusetzen. Hierbei spielt neben Langeweile häufig auch Klaustrophobie eine Rolle. Um diesem Problem zu begegnen, wurden im Stand der Technik bereits Konzepte vorgeschlagen, mittels denen die Erzeugung einer Bilddarstellung in dem Aufnahmebereich der Bildgebungseinrichtung erfolgt, in dem sich der Patient befindet. Hierdurch wird nicht nur der Langeweile des Patienten entgegengewirkt, sondern dieser wird zudem zur Überwindung der Klaustrophobie abgelenkt.

In der bislang unveröffentlichten deutschen Patentanmeldung mit dem amtlichen Aktenzeichen 10 2024 126 928.6 ist eine Bildgebungseinrichtung für eine Magnetresonanzvorrichtung beschrieben, mittels der Bilder und Videos, etwa ein Timer, eine Atemanleitung oder ein Film, an einer oberen Wand einer Magnetresonanzkammer der Magnetresonanzvorrichtung ausgebbar sind. Hierzu wird ein Bild über ein Bildübertragungsoptikfaserbündel zu einer Projektionsvorrichtung übertragen, die an einem Ende eines Tischkörpers eines Untersuchungstisches der Magnetresonanzvorrichtung angeordnet ist.

Weitere Konzepte zur Durchführung medizinischer Bildgebungsvorgänge, etwa mittels einer Magnetresonanztomographieeinrichtung, bei denen der Patient in einer Patientenaufnahme positioniert und mittels einer Darstellungsvorrichtung ein Bild innerhalb der Patientenaufnahme erzeugt wird, sind aus US 2024 / 0 045 004 A1, US 11 185 293 B2, US 5 627 902 A, US 2021 / 0 274 152 A1, US 5 877 732 A, US 9 939 500 B2 und US 9 661 313 B1 bekannt.

Die Erfindung stellt sich die Aufgabe, ein verbessertes Konzept bezüglich der Erzeugung einer Bilddarstellung in einem Aufnahmebereich einer Bildgebungseinrichtung anzugeben, in dem sich der Patient während der Durchführung der medizinischen Bildgebung befindet.

Erfindungsgemäß gelöst wird die Aufgabe bei einem Verfahren der eingangs genannten Art dadurch, dass die Bilddarstellung anhand der im Rahmen der Durchführung der Bildgebung erhobenen Bilddaten erfolgt.

Die Erfindung beruht auf dem Gedanken, dass es mittels der Erzeugung der Bilddarstellung anhand der Bilddaten ermöglicht wird, dem Patienten auf den Bildgebungsprozess gerichtete Informationen ausgeben zu können und diesem somit das Gefühl zu geben, aktiv in den Bildgebungsprozess mit eingebunden zu sein. So können die Bilddaten Handlungsanweisungen für den Patienten darstellen, beispielsweise bezüglich des Stillliegens und/oder eines Luftanhaltens. Weiterhin kann der, insbesondere medizinisch interessierte, Patient indem er bezüglich des Fortgangs des Bildgebungsprozesses konkret informiert wird, stärker motiviert werden, auch für längere Zeit still zu liegen, was der Bildqualität der Bilddaten zuträglich ist.

Der Aufnahmebereich kann röhrenförmig ausgebildet sein und in diesem Fall auch als ein Patiententunnel bezeichnet werden. Während der Durchführung der Bildgebung befindet sich der Patient häufig liegend auf einem in den Aufnahmebereich eingeschobenen Patiententisch. Dieser kann während der Durchführung der Bildgebung zur aktuell benötigten Positionierung des Patienten umpositioniert werden. Die Bilddarstellung wird in dem Aufnahmebereich erzeugt respektive ausgegeben. Das heißt, dass der Patient diese in seiner, insbesondere liegenden, Position erkennen kann, bevorzugt ohne hierbei seinen Kopf bewegen beziehungsweise anheben und/oder drehen muss. Die Ausgabe der Bilddarstellung erfolgt bevorzugt auf respektive über eine in oder an dem Aufnahmebereich angeordnete Darstellungsfläche, die ein Bildschirm respektive ein Display oder eine Projektionsfläche sein kann.

Bei der Bildgebungseinrichtung kann es sich um eine Magnetresonanztomographieeinrichtung handeln. Mittels einer Magnetresonanzeinrichtung können beispielsweise krankhafte Veränderungen im Körper, insbesondere Tumore, erkannt werden. Hierzu wird das Körperinnere des Patienten unter Einsatz von hohen Magnetfeldern Schicht für Schicht sichtbar gemacht. Zur Durchführung der Bildgebung werden Spins von Atomkernen des Körpers des Patienten mittels eines starken äußeren Magnetfeldes, das auch als Hauptmagnetfeld bezeichnet werden kann, ausgerichtet und mittels eines magnetischen Wechselfeldes zur Präzession um diese Ausrichtung angeregt. Die Präzession beziehungsweise Rückkehr der Spins aus diesem angeregten Zustand in einen Zustand mit geringerer Energie erzeugt als Antwort ein magnetisches Wechselfeld, das über Antennen empfangen wird.

Die Bilddaten können als wenigstens ein zweidimensionales Bild umfassend mehrere Pixel und/oder als wenigstens ein dreidimensionales Bild umfassend mehrere Voxel vorliegen. Das Bild bildet ein zwei- oder dreidimensionales Array von Pixeln oder Voxeln. Solche Arrays aus Pixeln oder Voxeln können Farbe, Intensität, Absorption oder andere Parameter als Funktion der zwei- oder dreidimensionalen Position repräsentieren. Sie können zum Beispiel durch geeignete Verarbeitung von Messsignalen der Bildgebungseinrichtung erhalten werden.

Bevorzugt umfasst die Bilddarstellung eine Schnittdarstellung des Teils des Körpers des Patienten, bezüglich dem während der Durchführung der Bildgebung die Bilddaten erfasst werden. Dem Patienten wird es so während der Durchführung der Bildgebung ermöglicht, in seinen eigenen Körper zu blicken und hierbei Strukturen wie Organe, Gefäße oder Knochen zu erkennen. Die dargestellte Schnittdarstellung zeigt denjenigen Bereich des Körpers des Patienten, für den auch aktuell im Zuge der Durchführung der Bildgebung die Bilddaten erhoben werden. Die Schnittdarstellung kann anhand der während der aktuellen Bildgebung bereits erfassten Bilddaten erzeugt werden respektive diese darstellen. Zudem oder alternativ kann die Schnittdarstellung anhand von Historiendaten, also beispielsweise anhand der Bilddaten eines früher durchgeführten Bildgebungsprozesses, erzeugt werden respektive diese darstellen.

Besonders bevorzugt entspricht die Schnittebene der Schnittdarstellung zumindest im Wesentlichen einer aktuellen Bildgebungsebene betreffend die Durchführung der Bildgebung. Hierdurch erfolgt eine noch bessere und unmittelbarere Einbindung des Patienten in den Bildgebungsprozess, da diesem in Echtzeit der Bereich oder die Schnittebene seines Körpers angezeigt wird, bezüglich dem oder der aktuell die Erhebung der Bilddaten erfolgt. Sofern während der Durchführung der Bildgebung Bilddaten betreffend mehrere Schnittebenen erhoben werden, erfolgt insbesondere eine Aktualisierung der aktuell dargestellten Schnittdarstellung an die jeweils aktuelle Bildgebungsebene.

Denkbar ist, dass die Schnittdarstellung auf der oder einer Darstellungsfläche, auf der die Bilddarstellung generiert wird, derart skaliert und positioniert ausgegeben wird, dass, bezogen insbesondere auf eine Längsrichtung der Bildgebungseinrichtung, die im Bereich der Schnittdarstellung vorliegenden Positionen zumindest im Wesentlichen den tatsächlich im Bereich des Körpers des Patienten entsprechen. Anders ausgedrückt entspricht eine Dimensionierung respektive Größe der Schnittdarstellung dem realen Bereich des Körpers des Patienten, der durch diese abgebildet wird. Eine Position eines auf der Schnittdarstellung dargestellten Objekts entspricht der realen Position dieses Objekts. Diese positionstreue Darstellung betrifft insbesondere die Längsrichtung der Bildgebungseinrichtung. Gemäß dieser Ausgestaltung entspricht folglich eine Längsposition eines auf der Schnittdarstellung dargestellten Objekts der realen Längsposition dieses Objekts. Die Längsrichtung kann einer Längsrichtung des, insbesondere röhrenförmigen, Aufnahmebereichs und/oder einer Hauptfeldrichtung der Magnetresonanztomographieeinrichtung entsprechen.

Zudem oder alternativ kann die Bilddarstellung wenigstens eine Informationsdarstellung umfassen, mittels der der Patient über wenigstens einen Umstand betreffend die aktuell durchgeführte Bildgebung informiert wird. Hierbei kann die Informationsdarstellung anhand der Bilddaten oder hieraus abgeleiteter Informationen erzeugt werden. Mittels der Informationsdarstellung werden dem Patient insbesondere Informationen betreffend einen aktuellen Fortschritt des Bildgebungsprozesses und/oder bereits im Zuge der Durchführung des Bildgebungsprozesses ermittelten Ergebnissen bereitgestellt.

Die wenigstens eine Informationsdarstellung kann die Schnittdarstellung, insbesondere zumindest teilweise, überlagern. So kann die Informationsdarstellung ein Informationsträger für konkrete Bereiche der Schnittdarstellung sein, wobei die überlagerte Informationsdarstellung über der Schnittdarstellung derart positioniert sein kann, dass die Position der Informationsdarstellung auf der Schnittdarstellung der Position desjenigen Bereichs der Schnittdarstellung entspricht, den diese Information betrifft. Die Informationsdarstellung kann teiltransparent sein, sodass der Bereich der Schnittdarstellung, an dem die Informationsdarstellung positioniert ist, für den Patienten sicht- und erkennbar bleibt. Zudem oder alternativ kann die Informationsdarstellung neben der Schnittdarstellung angeordnet sein.

Bevorzugt wird eine Segmentierung der Schnittdarstellung durchgeführt, bei der wenigstens eine auf der Schnittdarstellung dargestellte Körperstruktur des Patienten identifiziert wird, wobei die Informationsdarstellung eine Identifizierungsinformationsdarstellung ist, die den segmentierten Köperbereich und/oder die jeweils vorliegende Identifizierung angibt. Hierdurch wird der Patient konkret über auf der Schnittdarstellung erkennbare Strukturen informiert. Bei der Körperstruktur kann es sich um ein Organ, ein Gefäß oder einen Knochen beziehungsweise eine Knochenstruktur handeln. Die Identifizierungsinformationsdarstellung kann derart generiert werden, dass die segmentierte Körperstruktur, insbesondere flächig und/oder farbig, markiert ist. Zudem oder alternativ kann die in Identifizierungsinformationsdarstellung die Benennung der jeweiligen Körperstruktur in Textform umfassen.

Bevorzugt ist die wenigstens eine Informationsdarstellung wenigstens eine Zeitinformationsdarstellung, die einen zeitlichen Fortschritt der Durchführung der Bildgebung oder eines zeitlichen Teilintervalls der Bildgebung betrifft. Mittels der Zeitinformationsdarstellung wird der Patient hinsichtlich einer bereits verstrichenen und/oder noch anstehenden Zeitspanne informiert, die zur Durchführung der gesamten oder die jeweilige Schnittebene betreffenden Bildgebung vorgesehen beziehungsweise erforderlich ist.

Die wenigstens eine Zeitinformationsdarstellung kann eine konkrete Zahlenausgabe sein oder umfassen, die beispielsweise die bereits verstrichene oder noch verbleibende Zeit für die jeweilige Bildgebung angibt. Sofern die Bilddarstellung die Schnittdarstellung umfasst, ist bevorzugt vorgesehen, dass die wenigstens eine Zeitinformationsdarstellung einen bereits im Rahmen der Durchführung der Bildgebung abgescannten Bereich der Schnittdarstellung von einem im Rahmen der Durchführung der Bildgebung noch abzuscannenden Bereich der Schnittdarstellung abgrenzt. Dieser Vorgang kann beim Abcannen mehrerer Bildgebungsebenenen entsprechend mehrmals nacheinander wiederholt werden. Der abgescannte Bereich bezeichnet denjenigen Bereich der Schnittdarstellung, bezüglich dem bereits Bilddaten erfasst wurden. Der noch abzuscannende Bereich bezeichnet denjenigen Bereich der Schnittdarstellung, bezüglich dem die Bilddaten noch nicht erfasst wurden und noch zu erfassen sind. Insbesondere wenn die Schnittdarstellung anhand der Historiendaten erzeugt wird, kann der bereits abgescannte Bereich und der noch abzuscannende Bereich mittels wenigstens eines unterschiedlichen Darstellungsparameters angezeigt werden. Der Darstellungsparameter kann eine Helligkeit und/oder eine Schärfe der Bilddarstellung respektive der Schnittdarstellung betreffen. So kann der bereits abgescannte Bereich dunkler und/oder schärfer als der noch abzuscannende Bereich dargestellt werden. Insbesondere wenn die Schnittdarstellung anhand der Bilddaten erzeugt wird, kann der noch abzuscannende Bereich, für den noch keine Bilddaten vorliegen, leer, etwa ausgegraut, dargestellt werden.

Die wenigstens eine Zeitinformationsdarstellung kann ein die Schnittdarstellung überstreichender Balken oder eine die Schnittdarstellung überstreichende Linie sein. Der Balken beziehungsweise die Linie kann gerade sein. So wird mit fortschreitender Erhebung der Bilddaten der abgescannte Bereich immer größer und der noch abzuscannende Bereich immer kleiner. Der Balken oder die Linie wandert hierbei über die Schnittdarstellung. Konkret kann der Balken oder die Linie auf der Schnittdarstellung senkrecht auf der Längsrichtung stehen und/oder entlang der Längsrichtung wandern.

Insbesondere wenn die wenigstens eine Zeitinformationsdarstellung neben der Schnittdarstellung angeordnet ist, kann diese in Form einer Ampeldarstellung vorliegen. So kann zu Beginn der Bildgebung die Ampel auf Rot stehen, bei fortgeschrittener Zeitdauer auf Gelb und zum Ende der Bildgebung hin auf Grün wechseln. Zudem oder alternativ kann die Zeitinformationsdarstellung ein sich, insbesondere stetig, aufbauender Balken sein.

Denkbar ist, dass die wenigstens eine Wiedergabeeinrichtung eine außerhalb des Aufnahmebereichs angeordnete Bilderzeugungseinheit umfasst, mittels der Lichtstrahlen betreffend die Bilddarstellung generiert werden, wobei die Lichtstrahlen mittels einer Einkopplungseinheit in in den Aufnahmebereich führende Lichtleitfasern eingekoppelt werden, wobei die Lichtstrahlen mittels einer Auskopplungseinheit im Bereich des Aufnahmebereichs ausgekoppelt werden und die Bilddarstellung generieren. Insbesondere da in dem Aufnahmebereich häufig starke Magnetfelder vorliegen, aufgrund derer eine elektrische Signalübertragung ins Innere des Aufnahmebereichs nicht problemlos möglich ist, erfolgt im Rahmen dieser Ausführungsform die Signalübertragung ins Innere des Aufnahmebereichs mittels optischer Signale respektive Lichtstrahlen. Im Rahmen dieser Ausführungsform können die in der bereits eingangs genannten, deutschen Patentanmeldung mit dem amtlichen Aktenzeichen 10 2024 126 928.6 beschriebenen Aspekte und Merkmale realisiert sein.

Die Bilderzeugungseinheit ist beispielsweise ein Projektor, mittels dem elektrische Signale betreffend die Bilddarstellung in optische Signale umgewandelt werden. Die optischen Signale respektive Lichtstrahlen werden mittels der Einkopplungseinheit, die optische Elemente wie Linsen und/oder dergleichen umfassen kann, in die Lichtleitfasern eingekoppelt. Diese Einkopplung erfolgt über ein außerhalb des Aufnahmebereichs angeordnetes, stirnseitiges Ende der Lichtleitfasern. An dem diesem Ende gegenüberliegenden stirnseitigen Ende der Lichtleitfasern, das sich in oder an dem Aufnahmebereich befindet, erfolgt die Auskopplung der optischen Signale respektive Lichtstrahlen mittels der Auskopplungseinheit, die sodann zur Erzeugung der Bilddarstellung genutzt werden.

Bei den Lichtleitfasern handelt es sich bevorzugt um Glasfasern. Eine Anzahl der Lichtleitfasern kann zumindest 100.000, insbesondere zumindest 300.000 betragen. Insbesondere kann jede der Lichtleitfasern genau einem Pixel der Bilddarstellung zugeordnet sein, sodass jede der Lichtleitfasern denjenigen Lichtstrahl zur Erzeugung des jeweiligen Pixels führt. Die Lichtleitfasern können zu wenigstens einem Faserbündel zusammengefasst sein. Besonders bevorzugt sind die Lichtleitfasern positionsfest in wenigstens einem ortsfesten Abschnitt der Bildgebungseinrichtung angeordnet sind. Unter dem ortsfesten Abschnitt ist ein Bereich der Bildgebungseinrichtung zu verstehen, der im bestimmungsgemäßen Normalbetrieb der Bildgebungseinrichtung positionsfest bezüglich eines Untergrunds, auf dem die Bildgebungseinrichtung steht, verbleibt. Der ortsfeste Abschnitt kann beispielsweise ein den Aufnahmebereich tragendes Gestell und/oder Gehäuse und/oder eine den Aufnahmebereich bildende Struktur sein, die insbesondere die Spulen umfasst, die zur Erzeugung der zur Bildgebung erforderlichen Magnetfelder vorgesehen sind.

Die mittels der Auskopplungseinheit im Bereich des Aufnahmebereichs ausgekoppelten Lichtstrahlen können die Bilddarstellung als eine Projektion auf einer den Aufnahmebereich begrenzenden Wand generieren. Anders ausgedrückt werden die Lichtstrahlen mittels der Auskopplungseinheit in den Aufnahmebereich emittiert, sodass diese auf der Wand auftreffen und insgesamt die Bilddarstellung generieren. Die Wand bildet mithin die als die Projektionsfläche ausgebildete Darstellungsfläche. Die Wand kann eine Beschichtung aufweisen, mittels der etwa eine bezüglich eines Reflexionsgrades effektive und/oder bezüglich einer Abstrahlrichtung gleichmäßige Reflexion der Lichtstrahlen ermöglicht wird.

Insbesondere wenn der Aufnahmebereich röhrenförmig ist, ist die Wand, insbesondere kreisförmig, gebogen ausgebildet. Die geometrische Form der Wand kann zumindest ein Abschnitt eines geraden Kreiszylindermantels sein. Insbesondere im Fall der gebogenen Wand ist denkbar, dass eine Position der Bilddarstellung auf der Wand änderbar ist. So ist denkbar, dass eine, insbesondere zentrale, Tangentialebene der Schnittdarstellung parallel zu der aktuellen Bildgebungsebene verläuft. Konkret kann eine sich senkrecht zu der Längsrichtung erstreckende, insbesondere zentrale, Tangentialrichtung der Schnittdarstellung parallel zu einer sich senkrecht zu der Längsrichtung erstreckenden Richtung der aktuellen Bildgebungsebene verlaufen.

Die Auskopplungseinheit ist oder umfasst bevorzugt eine optische Umlenkeinheit, insbesondere eine Zerstreuerlinse oder Fischaugenlinse. So können die im Bereich der Auskopplungseinheit aus den Lichtleitfasern ausgekoppelten Lichtstrahlen im Wesentlichen parallel zueinander verlaufen, wobei die Ausbreitungsrichtungen der Lichtstrahlen mittels der zur Umlenkeinheit derart umgelenkt werden, dass diese auf die jeweils zur Erzeugung der Bilddarstellung erforderliche Position auf der Projektionsfläche auftreffen. Bevorzugt ist oder umfasst die Umlenkeinheit eine Fischaugenlinse. Mittels der Fischaugenlinse wird ein besonders großer Bildbereich abgedeckt. Das bedeutet, dass die mittels der Fischaugenlinse umgelenkten Lichtstrahlen einen Winkelbereich einnehmen, der beispielsweise mehr als 90°, bevorzugt mehr als 120°, besonders bevorzugt mehr als 180° beträgt.

Im Rahmen der vorliegenden Erfindung ist denkbar, dass sich die Bilddarstellung aus mehreren Teilbildern zusammensetzt. Die Teilbilder ergeben insgesamt die Bilddarstellung. So können mehrere Wiedergabeeinrichtungen vorgesehen sein oder die Wiedergabeeinrichtung kann mehrere Auskopplungseinheiten umfassen, mittels denen jeweils eines der Teilbilder generiert wird. Die Teilbilder können, insbesondere unmittelbar, benachbart entlang der Längsrichtung angeordnet sein. So können auch die Auskopplungseinheiten entlang der Längsrichtung verteilt angeordnet sein. Von der Einkopplungseinheit zu den Auskopplungseinheiten kann jeweils ein separates Faserbündel verlaufen, die abschnittsweise zu einem Gesamtbündel zusammengefasst sein können.

Die vorliegende Erfindung betrifft ferner eine Steuerungseinrichtung. Erfindungsgemäß gelöst wird die Aufgabe dadurch, dass diese eine Speichereinheit mit einem hierauf gespeicherten Computerprogramm umfassend ausführbare Anweisungen umfasst, die, wenn diese mittels einer Ausführungseinheit der Steuerungseinrichtung ausgeführt werden, die Ausführungseinheit dazu veranlassen, Steuerbefehle anhand der Bilddaten derart zu generieren und auszugeben, dass die Steuerbefehle die Wiedergabeeinrichtung zur Durchführung des Verfahrens gemäß der vorangehenden Beschreibung veranlassen. Alle im Zusammenhang mit dem erfindungsgemäßen Verfahren erläuterten Vorteile, Merkmale und Aspekte sind gleichermaßen auf die erfindungsgemäße Steuerungseinrichtung übertragbar und umgekehrt.

Die erfindungsgemäße Steuerungseinrichtung kann eine Komponente der Wiedergabeeinrichtung sein. In diesem Fall ist bevorzugt vorgesehen, dass die Bildgebungseinrichtung eine Steuereinheit mit einer Speichereinheit aufweist, seitens der die Bilddaten gespeichert werden und mithin vorliegen, wobei die Steuerungseinrichtung und die Steuereinheit zur Übertragung der Bilddaten entsprechend miteinander verbunden sind. Die Steuerungseinrichtung und die Speichereinheit können zu einer gemeinsamen Steuerungseinrichtung zusammengefasst sein. Insbesondere in diesem Fall ist die erfindungsgemäße Steuerungseinrichtung eine Komponente der Bildgebungseinrichtung.

Weiterhin betrifft die vorliegende Erfindung eine Wiedergabeeinrichtung zur Durchführung eines Verfahrens gemäß obiger Beschreibung, wobei mittels einer medizinischen Bildgebungseinrichtung während der Durchführung einer medizinischen Bildgebung Bilddaten betreffend zumindest einen Teil eines Körpers eines sich in einem Aufnahmebereich der Bildgebungseinrichtung befindenden Patienten erhebbar sind, wobei mittels der Wiedergabeeinrichtung eine Bilddarstellung für einen Patienten während der Durchführung einer medizinischen Bildgebung im Aufnahmebereich erzeugbar ist. Erfindungsgemäß gelöst wird die Aufgabe bei einer solchen Wiedergabeeinrichtung dadurch, dass diese eine Steuerungseinrichtung gemäß der vorangehenden Beschreibungspassage umfasst, mittels der die Steuerbefehle derart generierbar und ausgebbar sind, dass die Bilddarstellung anhand der im Rahmen der Durchführung der Bildgebung erhobenen Bilddaten erfolgt. Alle im Zusammenhang mit dem erfindungsgemäßen Verfahren und der erfindungsgemäßen Steuerungseinrichtung erläuterten Vorteile, Merkmale und Aspekte sind gleichermaßen auf die erfindungsgemäße Wiedergabeeinrichtung übertragbar und umgekehrt.

Die erfindungsgemäße Wiedergabeeinrichtung kann als ein Nachrüstset für eine bereits bestehende medizinische Bildgebungseinrichtung vorliegen. In diesem Fall umfasst die Wiedergabeeinrichtung die Steuerungseinrichtung und gegebenenfalls die Bilderzeugungseinheit, die Einkopplungseinheit, die Lichtleitfasern und die Auskopplungseinheit, die in die bereits bestehende Bildgebungseinrichtung zu verbauen sind.

Schließlich betrifft die vorliegende Erfindung eine medizinische Bildgebungseinrichtung zur Durchführung eines Verfahrens gemäß obiger Beschreibung, wobei mittels der Bildgebungseinrichtung während der Durchführung einer medizinischen Bildgebung Bilddaten betreffend zumindest einen Teil eines Körpers eines sich in einem Aufnahmebereich der Bildgebungseinrichtung befindenden Patienten erhebbar sind, wobei mittels einer Wiedergabeeinrichtung der Bildgebungseinrichtung eine Bilddarstellung für einen Patienten während der Durchführung einer medizinischen Bildgebung im Aufnahmebereich erzeugbar ist. Erfindungsgemäß gelöst wird die Aufgabe bei einer solchen Bildgebungseinrichtung dadurch, dass diese eine Steuerungseinrichtung gemäß der obigen Beschreibungspassage umfasst, mittels der die Steuerbefehle derart generierbar und ausgebbar sind, dass die Bilddarstellung anhand der im Rahmen der Durchführung der Bildgebung erhobenen Bilddaten erfolgt. Alle im Zusammenhang mit dem erfindungsgemäßen Verfahren, der erfindungsgemäßen Steuerungseinrichtung und der erfindungsgemäßen Wiedergabeeinrichtung erläuterten Vorteile, Merkmale und Aspekte sind gleichermaßen auf die erfindungsgemäße Bildgebungseinrichtung übertragbar und umgekehrt.

Bei der medizinischen Bildgebungseinrichtung kann es sich um eine Magnetresonanztomographieeinrichtung handeln. Die bereits im Zusammenhang mit dem erfindungsgemäßen Verfahren beschriebenen Aspekte der Magnetresonanztomographieeinrichtung sind auch auf die erfindungsgemäße Magnetresonanztomographieeinrichtung übertragbar.

Weitere Vorteile, Merkmale und Aspekte ergeben sich aus den nachfolgend dargelegten Ausführungsbeispielen sowie anhand der Figuren. Diese zeigen schematisch:
- Fig. 1:: eine schematische Ansicht auf eine erfindungsgemäße Bildgebungseinrichtung gemäß einem Ausführungsbeispiel, umfassend eine erfindungsgemäße Wiedergabeeinrichtung gemäß einem Ausführungsbeispiel mit einer erfindungsgemäßen Steuerungseinrichtung gemäß einem Ausführungsbeispiel, wobei anhand dieser Bildgebungseinrichtung ein erfindungsgemäßes Verfahren gemäß einem Ausführungsbeispiel erläutert wird,
- Fig. 2:: eine schematische Schnittdarstellung eines Aufnahmebereichs der Bildgebungseinrichtung der Fig. 1,
- Fig. 3, 4:: schematische Schnittdarstellungen des Aufnahmebereichs zur Veranschaulichung eines optionalen Aspekts der Erfindung,
- Fig. 5:: eine schematische, perspektivische Darstellung des Aufnahmebereichs zur Veranschaulichung einer möglichen Variante der Erfindung,
- Fig. 6:: eine schematische Ansicht auf eine Bilddarstellung sowie auf einen daneben dargestellten Patienten zur Veranschaulichung einer möglichen Variante der Erfindung,
- Fig. 7 - 9:: mehrere schematische Darstellungen einer zeitlichen Entwicklung einer im Rahmen der Durchführung des erfindungsgemäßen Verfahrens gemäß dem Ausführungsbeispiel erzeugten Bilddarstellung.

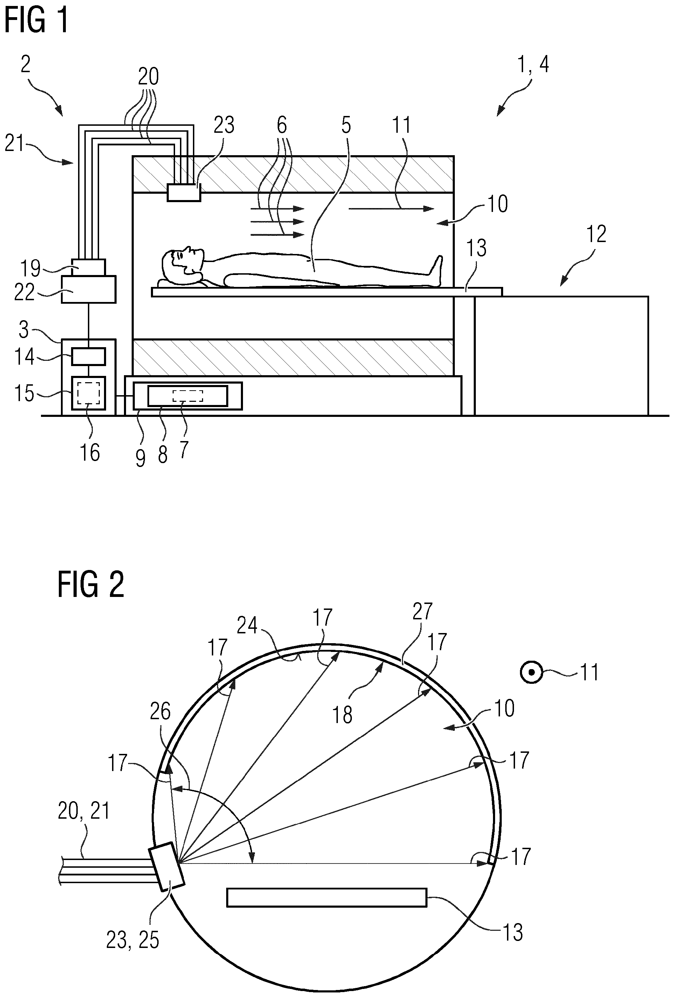

Fig. 1 zeigt eine seitliche, schematische Ansicht auf eine erfindungsgemäße medizinische Bildgebungseinrichtung 1 gemäß einem Ausführungsbeispiel. Diese umfasst eine erfindungsgemä-βe Wiedergabeeinrichtung 2 gemäß einem Ausführungsbeispiel mit einer erfindungsgemäßen Steuerungseinrichtung 3 gemäß einem Ausführungsbeispiel, wobei nachfolgend und anhand der in Fig. 1 gezeigten Komponenten ein erfindungsgemäßes Verfahren gemäß einem Ausführungsbeispiel erläutert wird.

Die Bildgebungseinrichtung 1 ist eine Magnetresonanztomographieeinrichtung 4, mittels der krankhafte Veränderungen im Körper eines Patienten 5 erkannt werden können. Hierzu wird das Körperinnere des Patienten 5 unter Einsatz hoher Magnetfelder 6, die mittels einer in den Figuren nicht gezeigten Magneteinrichtung erzeugt werden, sichtbar gemacht. Hierbei entstehen Bilddaten 7, die seitens einer Speichereinheit 8 einer Steuereinheit 9 der Bildgebungseinrichtung 1 gespeichert und in Echtzeit an die Steuerungseinrichtung 3 übertragen werden. Die Bilddaten 7 liegen als zwei- und/oder dimensionale Bilder vor.

Die Bildgebungseinrichtung 1 umfasst einen röhrenförmigen Aufnahmebereich 10 respektive Patiententunnel, der die geometrische Form eines geraden Kreiszylinders aufweist. Eine Längsrichtung 11 erstreckt sich entlang der Längsachse dieses Kreiszylinders sowie entlang einer Hauptfeldrichtung. Der Patient 5 kann mittels einer Patientenlagerungsvorrichtung 12 in den Aufnahmebereich 10 eingeschoben werden. Die Patientenlagerungsvorrichtung 12 weist hierzu einen in den Aufnahmebereich 10 hineinbewegbaren Patiententisch 13 auf.

Die Wiedergabeeinrichtung 2 ist mit einer als ein Computer ausgebildeten Ausführungseinheit 14 der Steuerungseinrichtung 3 verbunden, wobei seitens einer Speichereinheit 15 der Steuerungseinrichtung 3 ausführbare Anweisungen 16, also ein Computerprogramm, hinterlegt sind, die, wenn diese mittels der Ausführungseinheit 14 ausgeführt werden, diese dazu veranlassen, anhand der Bilddaten 7 Steuerbefehle zu generieren und an die Wiedergabeeinrichtung 2 auszugeben, sodass mittels dieser die Schritte des nachfolgend erläuterten Verfahrens durchgeführt werden.

Die Wiedergabeeinrichtung 2 umfasst eine außerhalb des Aufnahmebereichs 10 angeordnete Bilderzeugungseinheit 22, die vorliegend ein Projektor ist und mittels der anhand der Steuersignale und in Abhängigkeit der Bilddaten 7 Lichtstrahlen 17 zur Generierung einer später noch erläuterten Bilddarstellung 18 erzeugt werden. Die Lichtstrahlen 17 werden mittels einer Einkopplungseinheit 19 der Wiedergabeeinrichtung 2 in als Glasfasern vorgesehene Lichtleitfasern 20, die zu einem Faserbündel 21 zusammengefasst sind, eingekoppelt. Die Einkopplungseinheit 19 umfasst zu diesem Zweck in den Figuren nicht gezeigte optische Elemente wie beispielsweise Linsen und/oder dergleichen. Das Faserbündel 21 umfasst insgesamt wenigstens 100.000, nämlich beispielhaft 300.000, Lichtleitfasern 20. Die Komponenten der Wiedergabeeinrichtung 2 sind, obgleich dies in der Fig. 1 nicht konkret gezeigt ist, positionsfest in ortsfesten Abschnitten der Bildgebungseinrichtung 1 angeordnet.

Fig. 2 zeigt eine geschnittene Ansicht des Aufnahmebereichs 10 ohne den Patienten 5, wobei die Schnittebene senkrecht auf der Längsrichtung 11 steht. Die Lichtleitfasern 20 führen in den Aufnahmebereich 10, wo die Lichtstrahlen 17 mittels einer Auskopplungseinheit 23 der Wiedergabeeinrichtung 2 aus den Lichtleitfasern 20 ausgekoppelt und in den Aufnahmebereich 10 zur Erzeugung der Bilddarstellung 18 emittiert werden. Die ausgekoppelten Lichtstrahlen 17 erzeugen eine Projektion als die Bilddarstellung 18, und zwar auf einer den Aufnahmebereich 10 begrenzenden, beschichteten Wand 24, die eine Projektionsfläche bildet. Die Auskopplungseinheit 23 umfasst eine Fischaugenlinse 25, die bewirkt, dass die im Bereich der Auskopplungseinheit 23 aus den Lichtleitfasern 20 ausgekoppelten Lichtstrahlen 17, die parallel zueinander propagieren, derart umgelenkt werden, dass diese auf die zur Erzeugung der Bilddarstellung 18 erforderliche Position auf der Wand 24 auftreffen. Die mittels der Fischaugenlinse 25 umgelenkten Lichtstrahlen 17 umfassen einen Winkelbereich 26, der beispielhaft mehr als 90° beträgt.

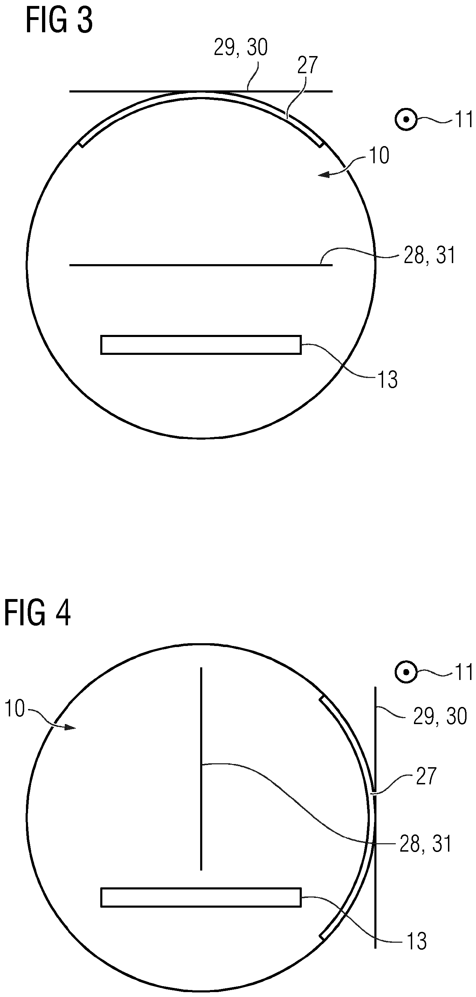

Vorliegend zeigt die Bilddarstellung 18 eine Schnittdarstellung 27 desjenigen Teils des Körpers des Patienten 5, bezüglich dem vorliegend die Bilddaten 7 erhoben werden. Die Schnittdarstellung 27 kann grundsätzlich anhand von Historiendaten erzeugt werden, die im Rahmen eines früheren Bildgebungsprozesses erhoben wurden. Auch kann die Schnittdarstellung 27 unmittelbar anhand der aktuellen Bilddaten 7 erzeugt werden. Eine Schnittebene der Schnittdarstellung 27 entspricht der Bildgebungsebene 28 betreffend die Durchführung der aktuellen Bildgebung. Hierbei hängt beispielhaft auch eine Position der Bilddarstellung 18 auf der Wand 24 von der Bildgebungsebene 28 ab, was nachfolgend anhand der Figuren 3 und 4 erläutert wird, die, gleichermaßen wie die Fig. 2, eine geschnittene Ansicht durch den Aufnahmebereich 10 zeigt. So ist vorgesehen, dass die Bilddarstellung 18 auf eine Position auf der Wand 24 derart projiziert wird, dass eine zentrale Tangentialebene 29 der Schnittdarstellung 27 parallel zu der aktuellen Bildgebungsebene 28 verläuft. Anders ausgedrückt verläuft eine sich senkrecht zu der Längsrichtung 11 erstreckende, zentrale Tangentialrichtung 30 der Schnittdarstellung 27 parallel zu einer sich senkrecht zu der Längsrichtung 11 erstreckenden Richtung 31 der aktuellen Bildgebungsebene 28.

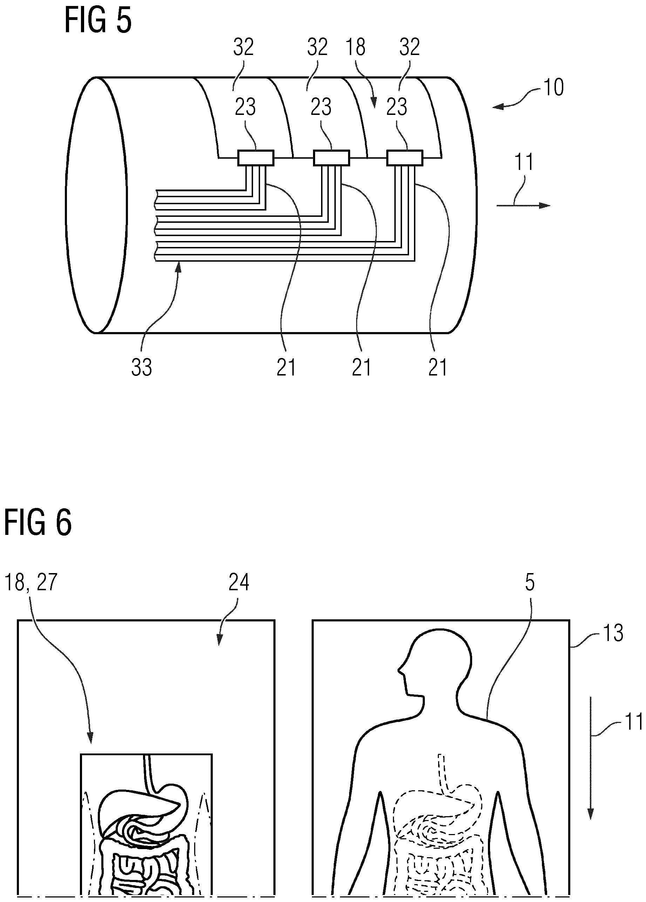

Nun wird auf die Fig. 5 zur Erläuterung einer alternativen Ausführungsvariante der vorliegenden Erfindung Bezug genommen, wobei diese Figur eine perspektivische Ansicht des Aufnahmebereichs 10 zeigt. Gemäß dieser Variante setzt sich die Bilddarstellung 18 aus mehreren, nämlich beispielhaft drei, Teilbildern 32 zusammen. Die Teilbilder 32 werden mittels jeweils einer Auskopplungseinheit 23 erzeugt. Die Teilbilder 32 sind unmittelbar benachbart entlang der Längsrichtung 11 angeordnet. Von der Einkopplungseinheit 19 zu den Auskopplungseinheiten 23 verläuft jeweils ein separates Faserbündel 21, die zu einem Gesamtbündel 33 zusammengefasst sind.

Ein weiterer denkbarer Aspekt bezüglich der vorliegenden Erfindung wird nachfolgend anhand der Fig. 6 erläutert. Diese Figur zeigt auf der linken Seite eine Ansicht von unten auf die Wand 24 mit der Schnittdarstellung 27 und auf der rechten Seite eine Ansicht von oben auf den Patiententisch 13 respektive den Patienten 5, wobei beide Seiten positionstreu bezüglich der Längsrichtung 11 sind. Aus Fig. 6 wird erkennbar, dass die Schnittdarstellung 27 derart skaliert und positioniert auf die Wand 24 projiziert wird, dass bezogen auf die Längsrichtung 11 die auf der Schnittdarstellung 27 vorhandenen Positionen den tatsächlichen Positionen im Bereich des Körpers des Patienten 5 entsprechen. Hierdurch wird es dem Patienten 5 ermöglicht, intuitiv das mittels der Schnittdarstellung 27 Gezeigte zu verstehen.

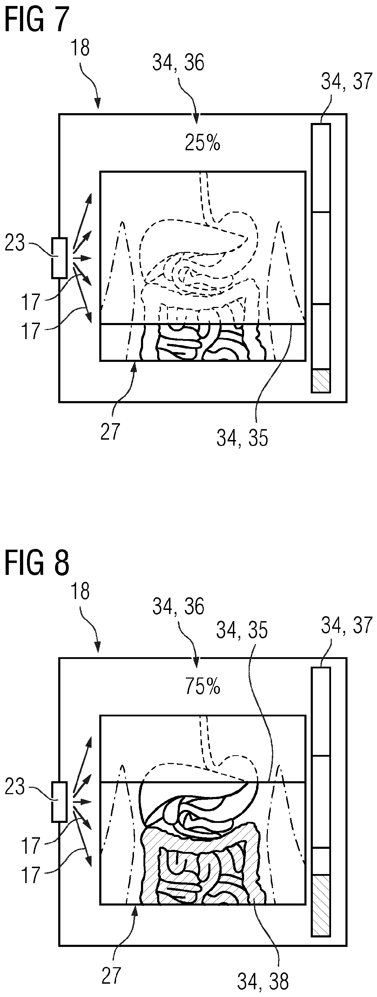

Nachfolgend wird auf die Figuren 7 - 9 Bezug genommen, die jeweils eine schematische Ansicht der Bilddarstellung 18 zeigen, und zwar wie sie im zeitlichen Verlauf während der Durchführung der Bildgebung generiert werden. Beispielhaft sei angenommen, dass vorliegend die Bildgebung bezüglich der Leber, des Magens und des Darms des Patienten 5 durchgeführt werden soll, wobei die Bilddaten 7 im Rahmen dreier hintereinander durchzuführenden Scans betreffend jeweils eine horizontale Bildgebungsebene 28 erfasst werden sollen, wobei jeder der Scans während einem entsprechenden zeitlichen Teilintervalls durchgeführt wird.

Die Figuren 7 - 9 betreffen die Durchführung des ersten Scans, also die Erhebung der Bilddaten 7 innerhalb der ersten der drei Bildgebungsebenen 28 während des ersten, zeitlichen Teilintervalls. Bezugnehmend auf die Fig. 7 umfasst die Bilddarstellung 18 gemäß dem bereits Erläuterten die Schnittdarstellung 27 betreffend die erste der drei Bildgebungsebenen 28. Anschließend und im Zuge der beiden nachfolgenden Scans sowie zeitlichen Teilintervalle erfolgt die Generierung der Bilddarstellung 18 analog zu den nachfolgend anhand des ersten Scans Erläuterten. Zusätzlich zu der Schnittdarstellung 27 umfasst die Bilddarstellung 18 mehrere Informationsdarstellungen 34, über die der Patient 5 über vorliegende Umstände betreffend die aktuell durchgeführte Bildgebung informiert wird.

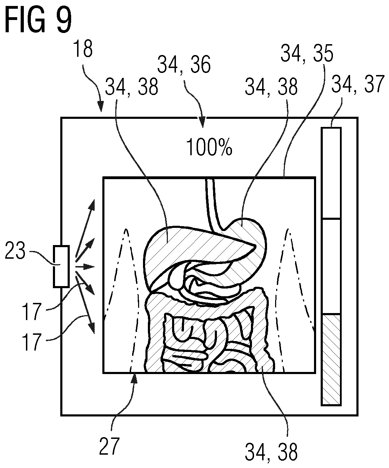

Eine der Informationsdarstellungen 34 ist eine erste Zeitinformationsdarstellung 35, die die Schnittdarstellung 27 überlagert. Die erste Zeitinformationsdarstellung 35 liegt in Form einer Linie oder eines Balkens vor, die oder der einen zeitlichen Fortschritt betreffend das jeweilige Teilintervall angibt. Konkret trennt die erste Zeitinformationsdarstellung 35 den im Rahmen der Durchführung dieses Scanvorgangs bereits abgescannten Bereich der Schnittdarstellung 27 von dem im Rahmen der Durchführung dieses Scanvorgangs noch abzuscannenden Bereich ab. In Fig. 7 ist in ein Viertel dieses Gesamtbereichs bereits abgescannt und mithin ein Viertel dieses Teilintervalls absolviert, wobei der unterhalb der ersten Zeitinformationsdarstellung 35 liegende Bereich der Schnittdarstellung 27 der bereits abgescannte Bereich und der oberhalb der ersten Zeitinformationsdarstellung 35 liegende Bereich der Schnittdarstellung 27 der noch abzuscannende Bereich ist. Der bereits abgescannte Bereich und der noch abzuscannende Bereich werden bezüglich der Schärfe und/oder der Helligkeit verschieden dargestellt. Vorliegend wird der noch abzuscannende Bereich heller und mit einer geringeren Schärfe als der bereits abgescannte Bereich dargestellt, was in den Figuren 7 - 9 durch gestrichelte Linien im noch abzuscannenden Bereich angedeutet ist. Sofern die Schnittdarstellung 27 anhand der Bilddaten 7 generiert wird, kann der noch abzuscannende Bereich, für den noch keine Bilddaten 7 vorliegen, leer, insbesondere ausgegraut, dargestellt werden. Anhand der Figuren 7 - 9 wird deutlich, dass die Zeitinformationsdarstellung 35 mit fortschreitender Dauer während des Bildgebungsprozesses immer weiter nach oben wandert, und zwar bis gemäß der Fig. 9 der oberen Rand des abzuscannenden Bereichs erreicht wurde und die Schnittdarstellung 27 nur noch den bereits abgescannten Bereich umfasst.

Eine weitere der Informationsdarstellungen 34 ist eine zweite Zeitinformationsdarstellung 36, die oberhalb der Schnittdarstellung 27 dargestellt wird. Die zweite Informationsdarstellung 36 gibt einen Zahlenwert in Prozent an, der die bereits verstrichene Dauer bezüglich des aktuellen Scanvorgangs respektive Teilintervallsangibt. Die erste Informationsdarstellung 35 und die zweite Informationsdarstellung 36 stellen quasi eine redundante Informationsausgabe dar.

Eine weitere der Informationsdarstellungen 34 ist eine dritte Zeitinformationsdarstellung 37, die seitlich neben der Schnittdarstellung 27 dargestellt wird. Die dritte Zeitinformationsdarstellung 37 realisiert einerseits eine Ampeldarstellung, andererseits einen sich mit fortschreitender Zeitdauer stetig aufbauenden Balken. Ersichtlich ist der Balken in drei gleich große Abschnitte aufgeteilt, wobei jeder dieser Abschnitte einem Scanvorgang betreffend eine der drei Bildgebungsebenen 28 und mithin einem der drei Teilintervalle zuordenbar ist. Die dritte Zeitinformationsdarstellung informiert den Patienten 5 bezüglich der bereits verstrichenen respektive noch anstehenden Gesamtdauer des gesamten Bildgebungsprozesses. Im Rahmen des anhand der Figuren 7 - 9 gezeigten, ersten Scanvorgangs baut sich der Balken innerhalb des untersten der drei Abschnitte auf. Die Ampeldarstellung wird dadurch realisiert, dass sich die Farbe des Balkens mit zunehmender Zeitdauer von Rot über Gelb zu Grün hin ändert.

Unter erneuter Bezugnahme auf die Figuren 7 - 9 wird schließlich ein weiterer Aspekt des vorliegenden Ausführungsbeispiels erläutert. So erfolgt während der Erhebung der Bilddaten 7 seitens einer auf der Steuereinheit 9 implementierten Auswertungssoftware eine Segmentierung der Bilddaten 7 respektive der Schnittdarstellung 27. Das bedeutet, dass auf der Schnittdarstellung 27 dargestellte Körperstrukturen identifiziert werden, und zwar hinsichtlich der zu untersuchenden Strukturen, also beispielsweise Organe und/oder Gefäße und/oder Knochen. Da für die vorliegende Bildgebung die Leber, der Magen und der Darm relevant sind, erfolgt eine entsprechende Segmentierung bezüglich dieser Körperstrukturen.

So sind weitere der Informationsdarstellungen 24 Identifizierungsinformationsdarstellungen 38, die die segmentierte Körperstruktur auf der Schnittdarstellung 27 kennzeichnen und hervorheben. Die Identifizierungsinformationsdarstellungen 38 überlagern die jeweiligen Körperstrukturen auf der Schnittdarstellung 27, wobei die verschiedenen Körperstrukturen mittels unterschiedlicher Farben kenntlich gemacht werden, was in den Figuren 8 und 9 durch unterschiedliche Schraffuren angedeutet ist. So erfolgte im Zuge der zur Generierung der in Fig. 8 gezeigten Schnittdarstellung 27 anhand der zu diesem Zeitpunkt vorliegenden Bilddaten 7 eine Identifizierung des Darms, während im Zuge der zur Generierung der in Fig. 9 gezeigten Schnittdarstellung 27 anhand der zu diesem Zeitpunkt vorliegenden Bilddaten 7 eine Identifizierung der Leber und des Magen erfolgte. Die Identifizierungsinformationsdarstellungen 38 sind teiltransparent, sodass die entsprechenden Bereiche der Schnittdarstellung 27 dennoch sichtbar bleiben. Obgleich dies vorliegend nicht konkret gezeigt ist, kann die Identifizierungsinformationsdarstellung 38 zusätzlich in Textform die jeweils im Rahmen der jeweiligen Segmentierung erkannte Struktur ausgeben, sodass beispielsweise neben der jeweiligen Identifizierungsinformationsdarstellung 38 der zugehörige Text "Leber", "Magen" beziehungsweise "Darm" angezeigt wird. Betreffend die Identifizierungsinformationsdarstellung 38 des Herzens kann diese auch ein animiertes Herz zeigen, das den Herzschlag des Patienten 5 visualisiert, etwa basierend auf Daten einer in Echtzeit erfolgenden Erfassung des Herzschlags des Patienten 5. Ergänzend wird angemerkt, dass bezüglich der Identifizierungsinformationsdarstellung 38, insbesondere in Echtzeit, die Verteilung eines Kontrastmittels im Körper respektive in Gefäßen des Patienten 5 dargestellt werden kann.

Durch die gemeinsame Darstellung der Schnittdarstellung 27 sowie der Informationsdarstellungen 34 wird für den Patienten 5 während der Durchführung des Bildgebungsprozesses und mittels der Bilddarstellung 18 eine sogenannte "Augmented Reality" erzeugt, die diesen umfangreich bezüglich des Bildgebungsprozesses informiert und diesen entsprechend einbindet.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Generierung einer Bilddarstellung (18) für einen Patienten (5) während der Durchführung einer medizinischen Bildgebung mittels einer medizinischen Bildgebungseinrichtung (1), wobei während der Durchführung der Bildgebung Bilddaten (7) betreffend zumindest einen Teil eines Körpers des sich in einem Aufnahmebereich (10) der Bildgebungseinrichtung (1) befindenden Patienten (5) erhoben werden, wobei mittels wenigstens einer Wiedergabeeinrichtung (2) die Bilddarstellung (18) für den Patienten (5) im Aufnahmebereich (10) generiert wird, **dadurch gekennzeichnet, dass** die Bilddarstellung (18) anhand der im Rahmen der Durchführung der Bildgebung erhobenen Bilddaten (7) erfolgt.

2. Verfahren nach Anspruch 1, **dadurch gekennzeichnet, dass** die Bilddarstellung (18) eine Schnittdarstellung (27) des Teils des Körpers des Patienten (5), bezüglich dem während der Durchführung der Bildgebung die Bilddaten (7) erfasst werden, umfasst.

3. Verfahren nach Anspruch 2, **dadurch gekennzeichnet, dass** die Schnittebene der Schnittdarstellung (27) zumindest im Wesentlichen einer aktuellen Bildgebungsebene (28) betreffend die Durchführung der Bildgebung entspricht.

4. Verfahren nach Anspruch 2 oder 3, **dadurch gekennzeichnet, dass** die Schnittdarstellung (27) auf einer Darstellungsfläche, auf der die Bilddarstellung (18) generiert wird, derart skaliert und positioniert ausgegeben wird, dass, bezogen insbesondere auf eine Längsrichtung (11) der Bildgebungseinrichtung (1), die im Bereich der Schnittdarstellung (27) vorliegenden Positionen zumindest im Wesentlichen den tatsächlich im Bereich des Körpers des Patienten (5) entsprechen.

5. Verfahren nach einem der vorangehenden Ansprüche, **dadurch gekennzeichnet, dass** die Bilddarstellung (18) wenigstens eine Informationsdarstellung (34) umfasst, mittels der der Patient (5) über wenigstens einen Umstand betreffend die aktuell durchgeführte Bildgebung informiert wird.

6. Verfahren nach einem der Ansprüche 2 bis 4 und nach Anspruch 5, **dadurch gekennzeichnet, dass** die wenigstens eine, insbesondere teiltransparente, Informationsdarstellung (34) die Schnittdarstellung (27) überlagert und/oder neben der Schnittdarstellung (27) angeordnet ist.

7. Verfahren nach Anspruch 6, **dadurch gekennzeichnet, dass** eine Segmentierung der Schnittdarstellung (27) durchgeführt wird, bei der wenigstens eine auf der Schnittdarstellung (27) dargestellte Körperstruktur des Patienten (5) identifiziert wird, wobei die Informationsdarstellung (34) eine Identifizierungsinformationsdarstellung (38) ist, die den segmentierten Köperbereich und/oder die jeweils vorliegende Identifizierung angibt.

8. Verfahren nach einem der Ansprüche 5 bis 7, **dadurch gekennzeichnet, dass** die wenigstens eine Informationsdarstellung (34) wenigstens eine Zeitinformationsdarstellung (35, 36, 37) ist, die einen zeitlichen Fortschritt der Durchführung der Bildgebung oder eines zeitlichen Teilintervalls der Bildgebung betrifft.

9. Verfahren nach einem der Ansprüche 2 bis 4 und nach Anspruch 8, **dadurch gekennzeichnet, dass** die wenigstens eine Zeitinformationsdarstellung (35, 36, 37) einen bereits im Rahmen der Durchführung der Bildgebung abgescannten Bereich der Schnittdarstellung (27) von einem im Rahmen der Durchführung der Bildgebung noch abzuscannenden Bereich der Schnittdarstellung (27) abgrenzt.

10. Verfahren nach Anspruch 9, **dadurch gekennzeichnet, dass** die wenigstens eine Zeitinformationsdarstellung (35, 36, 37) ein die Schnittdarstellung (27) überstreichender Balken oder eine die Schnittdarstellung (27) überstreichende Linie ist.

11. Verfahren nach einem der vorangehenden Ansprüche, **dadurch gekennzeichnet, dass** die wenigstens eine Wiedergabeeinrichtung (2) eine außerhalb des Aufnahmebereichs (10) angeordnete Bilderzeugungseinheit (22) umfasst, mittels der Lichtstrahlen (17) betreffend die Bilddarstellung (18) generiert werden, wobei die Lichtstrahlen (17) mittels einer Einkopplungseinheit (19) in in den Aufnahmebereich (10) führende Lichtleitfasern (20) eingekoppelt werden, wobei die Lichtstrahlen (17) mittels einer Auskopplungseinheit (23) im Bereich des Aufnahmebereichs (10) ausgekoppelt werden und die Bilddarstellung (18) generieren.

12. Verfahren nach Anspruch 11, **dadurch gekennzeichnet, dass** die mittels der, insbesondere als eine Fischaugenlinse (25) ausgebildeten, Auskopplungseinheit (23) im Bereich des Aufnahmebereichs (10) ausgekoppelten Lichtstrahlen (17) die Bilddarstellung (18) als eine Projektion auf einer den Aufnahmebereich (10) begrenzenden Wand (24) generieren.

13. Steuerungseinrichtung (3), umfassend eine Speichereinheit (15) mit einem hierauf gespeicherten Computerprogramm umfassend ausführbare Anweisungen (16), die, wenn diese mittels einer Ausführungseinheit (14) der Steuerungseinrichtung (3) ausgeführt werden, die Ausführungseinheit (14) dazu veranlassen, Steuerbefehle anhand der Bilddaten (7) derart zu generieren und auszugeben, dass die Steuerbefehle die Wiedergabeeinrichtung (2) zur Durchführung des Verfahrens nach einem der vorangehenden Ansprüche veranlassen.

14. Wiedergabeeinrichtung (2) zur Durchführung eines Verfahrens nach einem der Ansprüche 1 bis 12, wobei mittels einer medizinischen Bildgebungseinrichtung (1) während der Durchführung einer medizinischen Bildgebung Bilddaten (7) betreffend zumindest einen Teil eines Körpers eines sich in einem Aufnahmebereich (10) der Bildgebungseinrichtung (1) befindenden Patienten (5) erhebbar sind, wobei mittels der Wiedergabeeinrichtung (2) eine Bilddarstellung (18) für einen Patienten (5) während der Durchführung einer medizinischen Bildgebung im Aufnahmebereich (10) erzeugbar ist, **gekennzeichnet durch** eine Steuerungseinrichtung (3) nach Anspruch 13, mittels der die Steuerbefehle derart generierbar und ausgebbar sind, dass die Bilddarstellung (18) anhand der im Rahmen der Durchführung der Bildgebung erhobenen Bilddaten (7) erfolgt.

15. Medizinische Bildgebungseinrichtung (1) zur Durchführung eines Verfahrens nach einem der Ansprüche 1 bis 12, wobei mittels der Bildgebungseinrichtung (1) während der Durchführung einer medizinischen Bildgebung Bilddaten (7) betreffend zumindest einen Teil eines Körpers eines sich in einem Aufnahmebereich (10) der Bildgebungseinrichtung (1) befindenden Patienten (5) erhebbar sind, wobei mittels einer Wiedergabeeinrichtung (2) der Bildgebungseinrichtung (1) eine Bilddarstellung (18) für einen Patienten (5) während der Durchführung einer medizinischen Bildgebung im Aufnahmebereich (10) erzeugbar ist, **gekennzeichnet durch** eine Steuerungseinrichtung (3) nach Anspruch 13, mittels der die Steuerbefehle derart generierbar und ausgebbar sind, dass die Bilddarstellung (18) anhand der im Rahmen der Durchführung der Bildgebung erhobenen Bilddaten (7) erfolgt.
